# EUROPÄISCHE PATENTANMELDUNG

(11) **EP 3 957 349 A1**
(43) Veröffentlichungstag der Anmeldung: **23.02.2022**
(21) Anmeldenummer: 20191730.9
(22) Anmeldetag: 19.08.2020
(51) Int. Cl.: A61M 25/10

(54) **SCORINGBALLONKATHETER MIT INTRINSISCH FASEROPTISCHER DRUCK- UND TEMPERATURMESSUNG**

(71) Anmelder: Biotronik AG, 8180 Bülach (CH)
(72) Erfinder: SCHAEFER, Tobias, 78176 Blumberg-Fuetzen (DE)
(74) Vertreter: Biotronik Corporate Services SE

(57) **Zusammenfassung**

Die Erfindung betrifft einen Ballonkatheter (1) zum Aufweiten einer Stenose (S) in einem Gefäß (G) eines Patienten, mit: einem entlang einer Längsachse (x) erstreckten Katheterschaft (2), der einen distalen Endabschnitt (2a) aufweist, einen mit dem distalen Endabschnitt (2a) verbundenen Ballon (3), der eine nach außen gewandte Oberfläche (3a) sowie einen Balloninnenraum (30) aufweist, in den zum Inflatieren des Ballons (3) ein fluides Medium einleitbar ist, und mit zumindest einem Kontaktelement (40, 41, 42) bzw. Scoringelement, das auf der Oberfläche (3a) des Ballons (3) angeordnet ist und zum Aufweiten der Stenose (S) dazu ausgebildet ist, bei einem inflatierten Ballon (3) im Bereich der Stenose (S) gegen eine Gefäßwand (W) des Gefäßes (G) zu drücken. Erfindungsgemäß ist vorgesehen, dass das mindestens eine Kontaktelement (40, 41, 42) durch einen auf der Oberfläche (3a) des Ballons (3) angeordneten Lichtwellenleiter (41, 42) gebildet ist, der zur Temperatur- und/oder Druckmessung vorgesehen ist.

## Beschreibung

Die Erfindung betrifft einen Ballonkatheter gemäß Anspruch 1.

Ein derartige Ballonkatheter wird auch als Scoring-Ballonkatheter bezeichnet und weist einen Ballon auf, auf dessen nach außen gewandter Oberfläche zumindest ein längserstrecktes Kontaktelement festgelegt ist, das auch als Scoringelement bezeichnet wird.

Diese Kontaktelemente werden beim Inflatieren des Ballons innerhalb einer Stenose in die Stenose gedrückt und erlauben auf diese Weise ein mechanisches Öffnen bzw. ein Aufweiten der Stenose. Da die Kontaktelemente die Gefäßwandung bzw. Läsion im Bereich der Stenose beim Inflatieren des Ballons in der Regel zuerst kontaktieren, kann über die Kontaktelemente aufgrund der geringeren Oberfläche der Kontaktelemente im Vergleich zur Gesamtoberfläche des Ballons ein erhöhter mechanischer Druck in die Gefäßwandung bzw. Läsion eingeleitet werden, um diese Einzukerben. Hierdurch lässt sich die Stenose einfacher aufweiten bzw. entfernen.

Aus der US 7,329,223 ist eine intravaskuläre Vorrichtung zur Durchführung einer therapeutischen Behandlung bekannt, die mindestens eine Glasfaser aufweist, die dazu konfiguriert ist, diagnostische Informationen bereitzustellen. Die Glasfaser kann dabei beim Ballonformen auf die äußere Oberfläche des Ballons der Vorrichtung aufgebracht werden.

Weiterhin offenbart die US 10,258,240 einen Führungsdraht mit einem optischen Sensor für eine Druck- und Temperaturmessung.

Schließlich beschreibt die US 2016/0220131 ein Sensorsystem zur Messung von Druck und Temperatur in einem Blutgefäß mittels Rayleigh-Streuung.

Weiterhin sind aus dem Stand der Technik Scoringelemente bekannt, die durch ein Drahtgeflecht gebildet sind oder durch einzelne mit der Balloberfläche verklebte Elemente. Derartige Drahtelemente sind jedoch oftmals zu steif bzw. weisen einen hohen Querschnitt auf und sind daher nachteilig im Handling während der Aufweitung der Stenose. Weiterhin besteht bei einzelnen auf die Ballonoberfläche aufgeklebten Elementen prinzipiell das Risiko eines Verlustes eines solchen Elementes und damit einhergehend eine Verletzungsgefahr für den Patienten.

Hiervon ausgehend liegt der vorliegenden Erfindung daher die Aufgabe zugrunde, einen Ballonkatheter bereitzustellen, der das Einkerben von Läsionen im Bereich einer Stenose eines Gefäßes sowie eine Temperatur- und/oder Druckmessung während der Aufweitung der Stenose ermöglicht. Insbesondere soll der Ballonkatheter möglichst flexibel sein und einen geringen Querschnitt aufweisen, so dass der Ballonkatheter möglichst eine gute Einführbarkeit (Trackability) aufweist.

Diese Aufgabe wird durch einen Ballonkatheter mit den Merkmalen des Anspruchs 1 gelöst.

Vorteilhafte Ausgestaltungen sind in den Unteransprüchen angegeben und werden nachfolgend beschrieben.

Gemäß Anspruch 1 wird ein Ballonkatheter zum Aufweiten einer Stenose eines Gefäßes eines Patienten offenbart, mit:
- einem insbesondere entlang einer Längsachse erstreckten Katheterschaft, der einen distalen Endabschnitt aufweist,
- einem mit dem distalen Endabschnitt verbundenen Ballon, der eine nach außen gewandte Oberfläche sowie einen Balloninnenraum aufweist, in den zum Entfalten des Ballons ein fluides Medium einleitbar ist,
- zumindest einem Kontaktelement (auch als Scoringelement bezeichnet), das auf der Oberfläche des Ballons festgelegt ist und zum Aufweiten der Stenose dazu ausgebildet ist, bei einem entfalteten Ballon im Bereich der Stenose gegen die Gefäßwand bzw. Läsion zu drücken, um z.B. eine Einkerbung der Gefäßwand bzw. Läsion zu erreichen.

Erfindungsgemäß ist nun vorgesehen, dass das mindestens eine Kontaktelement durch einen auf der Oberfläche festgelegten sowie vorzugsweise längserstreckten Lichtwellenleiter gebildet ist, der zur Temperatur- und/oder Druckmessung konfiguriert ist. Längserstreckt bedeutet hierbei, dass der Lichtwellenleiter eine Länge entlang der Längsachse des Ballonkatheters aufweist, die um ein Vielfaches größer ist als ein Durchmesser des Lichtwellenleiters. Zurzeit weisen Lichtwellenleiter typischerweise einen Durchmesser zwischen 0.125 mm und 1 mm auf.

Wenn ferner im Rahmen der vorliegenden Erfindung von einer proximalen Komponente oder einem proximalen Bereich und einer entsprechenden distalen Komponente bzw. einem distalen Bereich die Rede ist, so bedeutet das, dass die distale Komponente bzw. der distale Bereich entlang des Verlaufs des Katheterschafts weiter von einem Bediener des Ballonkatheters entfernt ist, als die proximale Komponente bzw. der proximale Bereich. Bei dem Bereich kann es sich z.B. um einen Endabschnitt oder ein Ende einer Komponente des Ballonkatheters handeln.

Gemäß einer Ausführungsform der Erfindung ist vorgesehen, dass sich der mindestens eine Lichtwellenleiter zumindest abschnittsweise helixförmig, d.h. einer Schraubenlinie folgend, um den Ballon herum erstreckt. Dabei kann sich (z.B. bedingt durch die Geometrie des Ballons im entfalteten bzw. inflatierten Zustand) ein Radius und/oder eine Ganghöhe der Helix bzw. der Schraubenlinie ändern. Gemäß einer Ausführungsform weist der Ballon in einem inflatierten bzw. entfalteten Zustand einen zylindrischen Abschnitt auf, um den sich der mindestens eine Lichtwellenleiter mit einem konstanten Radius und/oder einer konstanten Ganghöhe helixförmig herum erstrecken kann.

Weiterhin ist gemäß einer Ausführungsform der Erfindung vorgesehen, dass der mindestens eine Lichtwellenleiter eine optische Faser aufweist oder durch eine optische Faser gebildet ist.

Weiterhin ist gemäß einer Ausführungsform der Erfindung vorgesehen, dass die optische Faser aus einem Glas, insbesondere einem Quarzglas, Fluoridglas oder Chalkogenidglas, oder aus einem Kunststoff (z.B. einem Polymer) gebildet ist. Bei der optischen Faser kann es sich also um eine Glasfaser oder eine polymere optische Faser handeln. In Frage kommen auch Fasern aus kristallinen Materialien wie z.B. Saphir.

Weiterhin ist gemäß einer Ausführungsform der Erfindung vorgesehen, dass der Ballonkatheter eine distale Katheterspitze aufweist, wobei sich der Ballon ausgehend vom distalen Endabschnitt des Katheterschafts zur Katheterspitze erstreckt. Weiterhin ist gemäß einer Ausführungsform der Erfindung vorgesehen, dass der Ballonkatheter einen im Katheterschaft angeordneten Innenschaft zur Aufnahme eines Führungsdrahtes des Ballonkatheters aufweist, wobei sich der Innenschaft durch den Balloninnenraum hindurch zur Katheterspitze erstreckt und insbesondere mit dieser verbunden ist. Die Katheterspitze bildet somit einen distalen Endabschnitt des Ballonkatheters, der insbesondere außerhalb des Balloninnenraumes entlang der Längsachse des Ballonkatheters verläuft, und weist vorzugsweise einen Ausgang für den Führungsdraht am distalen Ende des Ballonkatheters auf.

Weiterhin ist gemäß einer Ausführungsform der Erfindung vorgesehen, dass der mindestens eine Lichtwellenleiter ein distales Ende aufweist, das an der Katheterspitze festgelegt ist. Alternativ hierzu kann das distale Ende an einem distalen Ballonende des Ballons oder am Innenschaft festgelegt sein.

Weiterhin ist gemäß einer Ausführungsform der Erfindung vorgesehen, dass sich der mindestens eine Lichtwellenleiter (z.B. ausgehend von der Katheterspitze) entlang des Ballons sowie entlang des Katheterschafts zu einem proximalen Endabschnitt des Katheterschafts hin erstreckt.

Weiterhin ist gemäß einer Ausführungsform der Erfindung vorgesehen, dass der Ballonkatheter eine mit dem mindestens einen Lichtwellenleiter verbindbare Auswertungseinheit aufweist, wobei die Auswertungseinheit dazu ausgebildet ist, Licht (z.B. Laserlicht) in den mindestens einen Lichtwellenleiter einzukoppeln und durch Auswertung des durch den mindestens einen Lichtwellenleiters zurückgestreuten Lichts eine Temperatur und/oder einen Druck, insbesondere im Bereich des Ballons, zu bestimmen. Die Auswertungseinheit ist insbesondere dazu ausgebildet, die Temperatur bzw. den Druck entlang des Ballons ortsaufgelöst zu bestimmen.

Weiterhin kann gemäß einer Ausführungsform am proximalen Endabschnitt des Katheterschafts ein Konnektor vorgesehen sein, insbesondere ein Luer-Konnektor, an den die Auswertungseinheit anschließbar ist, so dass die Auswertungseinheit im mit dem Konnektor verbundenen Zustand Licht in den mindestens einen Lichtwellenleiter einkoppeln kann.

Die Auswertungseinheit kann zur Bestimmung der Temperatur und/oder des Drucks in bekannter Weise Faser-Bragg-Gitter des mindestens einen Lichtwellenleiters oder eine Messung der Rayleigh-Streuung im mindestens einen Lichtwellenleiter verwenden oder ein anderes bekanntes Verfahren.

Weiterhin ist gemäß einer Ausführungsform der Erfindung vorgesehen, dass der Katheterschaft ein mit dem Balloninnenraum strömungstechnisch verbundenes Lumen aufweist, über das das fluide Medium zum Inflatieren/Entfalten des Ballons in den Balloninnenraum einleitbar ist. Mittels des Mediums kann der Druck eingestellt werden, mit dem das mindestens eine Kontakt- bzw. Scoringelement im Bereich der Stenose gegen die Gefäßwandung/Läsion drückt.

Weiterhin ist gemäß einer Ausführungsform der Erfindung vorgesehen, dass der Ballonkatheter mehrere Kontaktelemente bzw. Scoringelemente aufweist, die auf der Oberfläche des Ballons festgelegt sind und zum Aufweiten der Stenose dazu ausgebildet sind, bei einem entfalteten Ballon im Bereich der Stenose gegen die Gefäßwand (bzw. Läsion) zu drücken, um z.B. eine Einkerbung der Gefäßwandung/Läsion zu erreichen, wobei das jeweilige Kontaktelement durch einen auf der Oberfläche festgelegtem sowie vorzugsweise längs erstreckten Lichtwellenleiter gebildet ist, wobei der jeweilige Lichtwellenleiter zur Temperatur- und/oder Druckmessung konfiguriert ist.

Weiterhin ist gemäß einer Ausführungsform der Erfindung vorgesehen, dass der Ballonkatheter zwei bis fünf als Lichtwellenleiter ausgebildete Kontaktelemente aufweist. Gemäß einer bevorzugten Ausführungsform weist der Ballonkatheter drei als Lichtwellenleiter ausgebildete Kontaktelemente auf.

Weiterhin ist gemäß einer Ausführungsform der Erfindung vorgesehen, dass sich der jeweilige Lichtwellenleiter zumindest abschnittsweise helixförmig (d.h. einer Schraubenlinie folgend) um den Ballon herum erstreckt.

Der jeweilige Lichtwellenleiter kann wiederum eine optische Faser aufweisen oder durch eine optische Faser gebildet sein. Die jeweilige Faser kann aus einem Glas (insbesondere einem Quarzglas) oder einem Kunststoff (z.B. einem Polymer) gebildet sein.

Der jeweilige Lichtwellenleiter kann weiterhin ein distales Ende aufweisen, das an der Katheterspitze festgelegt ist, wobei sich der jeweilige Lichtwellenleiter insbesondere entlang des Ballons sowie entlang des Katheterschafts zu dem proximalem Endabschnitt des Katheterschafts erstrecken kann. Alternativ kann das distale Ende des jeweiligen Lichtwellenleiters an einem distalen Ballonende oder am Innenschaft festgelegt sein.

Bei mehreren Lichtwellenleitern ist die Auswertungseinheit insbesondere dazu ausgebildet, Licht (z.B. Laserlicht) in den jeweiligen Lichtwellenleiter einzukoppeln und durch Auswertung des durch den jeweiligen Lichtwellenleiters zurückgestreuten Lichts eine Temperatur und/oder einen Druck, insbesondere im Bereich des Ballons, zu bestimmen, insbesondere ortsaufgelöst (siehe oben). Die Auswertungseinheit kann zur Bestimmung der Temperatur und/oder des Drucks unterschiedliche Lichtwellenleiter (sofern vorhanden) verwenden.

Weitere Merkmale und Vorteile der Erfindung sollen bei der Figurenbeschreibung von Ausführungsbeispielen der Erfindung anhand der Figur erläutert werden. Es zeigt:

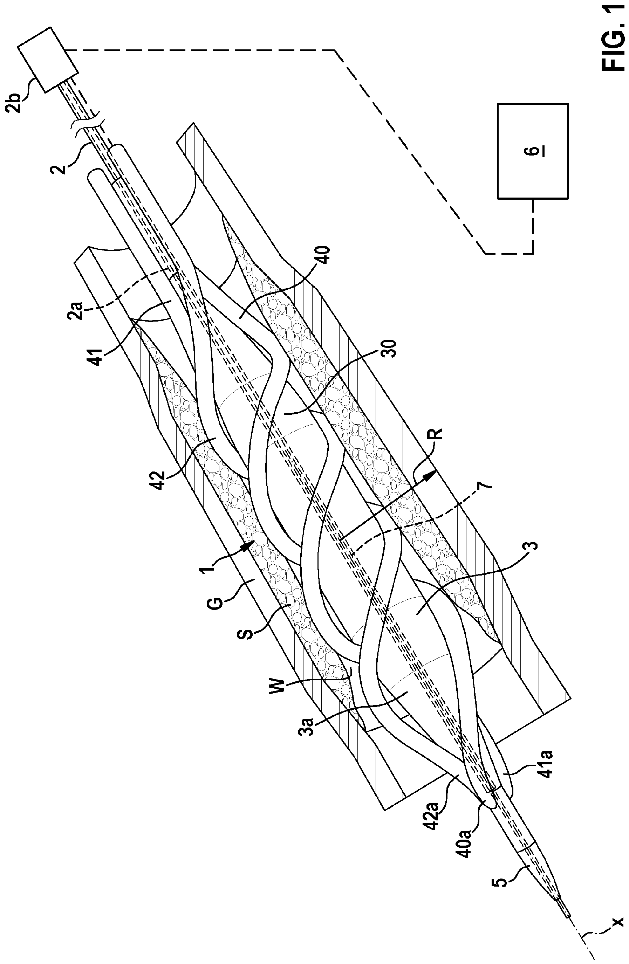

- Fig. 1: eine perspektivische Ansicht einer Ausführungsform eines erfindungsgemäßen Ballonkatheters.

Die Figur 1 zeigt eine Ausführungsform eines erfindungsgemäßen Ballonkatheters 1, der zum Aufweiten einer Stenose S in einem Gefäß G eines Patienten dient. Der Ballonkatheter 1 weist hierzu einen flexiblen, entlang einer Längsachse x erstreckten Katheterschaft 2 auf, der sich von einem proximalen Endabschnitt 2b hin zu einem distalen Endabschnitt 2a erstreckt, der mit einem Ballon 3 verbunden ist, der eine nach außen gewandte Oberfläche 3a aufweist und einen Balloninnenraum 30 umgibt, in den zum Inflatieren des Ballons 3 ein fluides Medium einleitbar ist. Weiterhin weist der Ballon 3 vorliegend exemplarisch drei Kontaktelemente 40, 41, 42 auf, die auch als Scoringelemente 40, 41, 42 bezeichnet werden. Die Scoringelemente 40, 41, 42 sind auf der Oberfläche 3a des Ballons 3 angeordnet und befestigt so dass sie bei einem inflatierten Ballon 3 im Bereich der Stenose S gegen eine Gefäßwand W des Gefäßes G drücken um die Stenose S aufzuweiten, z.B. durch Erzeugung einer Einkerbung in der verengten Gefäßwand W.

Die Scoringelemente 40, 41, 42 sind dabei jeweils durch einen längs erstreckten Lichtwellenleiter 41, 42 gebildet, wobei die Lichtwellenleiter 40, 41, 42 neben ihrer Funktion als Scoringelemente zur Temperatur- und/oder Druckmessung konfiguriert sind.

Die Lichtwellenleiter 40, 41, 42 erstrecken sich gemäß Figur 1 vorzugsweise helixförmig in Umfangsrichtung um den Ballon 3 herum, wobei sie sich auch entlang der Längsachse x erstrecken, wenn der Ballon 3 seinen inflatierten Zustand einnimmt. Dieser Zustand wird hergestellt, indem das fluide Medium in den Balloninnenraum 30 eingeleitet wird, so dass sich der Ballon 3 in radialer Richtung R entfaltet (senkrecht zur Längsachse x) und dabei ausdehnt.

Die Lichtwellenleiter 40, 41, 42 sind vorzugsweise distal an einer Katheterspitze 5 bzw. am Ballon 3 oder an einem Innenschaft 7 des Ballonkatheters 1 fixiert. Hierzu können z.B. die distalen Enden 40a, 41a, 42a der Lichtwellenleiter 40, 41, 42 an der Katheterspitze 5 festgelegt sein. Die distalen Enden 40a, 41a, 42a der Lichtwellenleiter 40, 41, 42 werden dabei vorzugsweise zusammen mit dem distalen Ende des Innenschaftes 7 und dem distalen Ende des distalen Ballonhalses (nicht im Detail dargestellt) zu einer integralen Katheterspitze 5 verschmolzen. Der im Katheterschaft 2 angeordneten Innenschaft 7 dient vorzugsweise zur Aufnahme eines Führungsdrahtes des Ballonkatheters 1 und erstreckt sich insbesondere entlang der Längsachse x durch den Balloninnenraum 30 hindurch zur Katheterspitze 5, so dass der Führungsdraht über die Katheterspitze 5 aus dem Innenschaft 7 herausführbar ist.

Ab der proximalen Ballonseite hin zum proximalen Endabschnitt 2b des Katheterschafts 2 sind die Lichtwellenleiter 40, 41, 42 entlang des Schaftes 2 geführt und enden proximal z.B. in einem Konnektor 2b, der als Luer-Konnektor ausgestaltet sein kann. Am Luer 2b ist vorzugsweise eine zusätzliche Anschlussmöglichkeit für eine vorhandene Auswertungseinheit 6 vorgesehen.

Die Lichtwellenleiter 40, 41, 42 dienen in erster Linie als Scoringelemente. Die ausgezeichnete Biegsamkeit der Lichtwellenleiter (z.B. optischer Faser, insbesondere Glasfaser oder polymere optische Faser) 40, 41, 42 lässt eine verbesserte Einführbarkeit (Trackability) im Vergleich zu z.B. Nitinol-Scoringelementen zu. Die hohe Festigkeit der Lichtwellenleiter 40, 41, 42 unterstützt weiterhin eine zuverlässige Einkerbung in der Stenose S.

Mit Vorteil dienen die Lichtwellenleiter 40, 41, 42 gleichzeitig als Sensor sowie als Signalleitung, über die die Sensordaten zu der Auswertungseinheit 6 übertragen werden (z.B. in Form des zurückgestreuten Lichts). Im Vergleich zu anderen Sensoren liegt somit insbesondere ein einteiliges System ohne Sensor/Leitung-Schnittstelle vor. Die Vorteile der Lichtwellenleiter 40, 41, 42, liegen vor allem in ihrer Robustheit gegen äußere Einflüsse (z.B. elektromagnetische Felder).

Z.B. durch den Raman-Effekt bzw. die Rayleigh-Streuung, kann die Temperatur in der Stenose S oder auch einfach in der Arterie (z.B. bei entzündeten Stellen) durch einen geeigneten Algorithmus errechnet werden, der z.B. durch die Auswertungseinheit ausgeführt wird. Somit sind je Lichtwellenleiter 40, 41, 42, insbesondere eine räumliche Auflösung von 1 mm und eine Temperaturauflösung von 0,1 °C möglich.

Der beim Öffnen der Stenose S entstehende Druck der Gefäß-/Stenosenwand W auf die Lichtwellenleiter 40, 41, 42 induziert Biegeverluste und führt somit zu Transmissionsänderungen, die wiederum durch einen geeigneten Algorithmus, der durch die Auswertungseinheit 6 ausgeführt wird, gemessen und verarbeitet werden können.

Durch die helixförmige Wicklung der Lichtwellenleiter 40, 41, 42 um den Ballon 3 herum sind mit Vorteil Messungen über den ganzen Umfang des Gefäßes G bzw. der Stenose S möglich.

Die erfindungsgemäßen Lichtwellenleiter 40, 41, 42 kombinieren also gleich mehrere Funktionen: mechanische Kraftausübung als Scoringelemente, Temperaturmessung, Druckmessung, und Datenübertragung.

Diese Funktionsintegration ermöglicht in vorteilhafter Weise vergleichsweise geringe Kosten in der Herstellung, insbesondere durch Verarbeitung günstiger und bekannter Materialien (z.B. Glasfasern). Die Verwendung von Lichtwellenleitern als Sensoren erweist sich als sehr robust. Insbesondere die oftmals kritische Datenübertragung (z.B. bei metallischen Leitern) vom Sensor zur Auswertungseinheit kann auf einfache Weise mittels der Lichtwellenleiter bereitgesellt werden. Schließlich gewährleistet die Verwendung von Lichtwellenleitern eine vergleichsweise gute Einführbarkeit bzw. ein zufriedenstellendes Biegemoment des Ballonkatheters.

## Patentansprüche

1. Ballonkatheter (1) zum Aufweiten einer Stenose (S) in einem Gefäß (G) eines Patienten, mit:
- einem Katheterschaft (2), der einen distalen Endabschnitt (2a) aufweist,
- einem mit dem distalen Endabschnitt (2a) verbundenen Ballon (3), der eine nach außen gewandte Oberfläche (3a) sowie einen Balloninnenraum (30) aufweist, in den zum Inflatieren des Ballons (3) ein fluides Medium einleitbar ist,
- zumindest einem Kontaktelement (40, 41, 42), das auf der Oberfläche (3a) des Ballons (3) angeordnet ist und zum Aufweiten der Stenose (S) dazu ausgebildet ist, bei einem inflatierten Ballon (3) im Bereich der Stenose (S) gegen eine Gefäßwand (W) des Gefäßes (G) zu drücken,
**dadurch gekennzeichnet,**
**dass** das mindestens eine Kontaktelement (40, 41, 42) durch einen auf der Oberfläche (3a) des Ballons (3) angeordneten Lichtwellenleiter (41, 42) gebildet ist, der zur Temperatur- und/oder Druckmessung vorgesehen ist.

2. Ballonkatheter nach Anspruch 1, **dadurch gekennzeichnet, dass** sich der mindestens eine Lichtwellenleiter (41, 42) bei einem inflatierten Ballon (3) helixförmig um den Ballon (3) herum erstreckt.

3. Ballonkatheter nach Anspruch 1 oder 2, **dadurch gekennzeichnet, dass** der mindestens eine Lichtwellenleiter (41, 42) eine optische Faser aufweist oder durch eine optische Faser (41, 42) gebildet ist.

4. Ballonkatheter nach Anspruch 3, **dadurch gekennzeichnet, dass** die optische Faser aus einem Glas oder aus einem Kunststoff gebildet ist.

5. Ballonkatheter nach einem der vorhergehenden Ansprüche, **dadurch gekennzeichnet, dass** der Ballonkatheter (1) eine distale Katheterspitze (5) aufweist, wobei sich der Ballon (3) ausgehend vom distalen Endabschnitt (2a) des Katheterschafts (2) zur Katheterspitze (5) erstreckt.

6. Ballonkatheter nach Anspruch 5, **dadurch gekennzeichnet, dass** der mindestens eine Lichtwellenleiter (40, 41, 42) ein distales Ende (40a, 41a. 42a) aufweist, das an der Katheterspitze (5) festgelegt ist.

7. Ballonkatheter nach Anspruch 5 oder 6, **dadurch gekennzeichnet, dass** sich der mindestens eine Lichtwellenleiter (40, 41, 42) ausgehend von der Katheterspitze (5) entlang des Ballons (3) sowie entlang des Katheterschafts (2) zu einem proximalem Endabschnitt (2b) des Katheterschafts (2) hin erstreckt.

8. Ballonkatheter nach einem der vorhergehenden Ansprüche, **dadurch gekennzeichnet, dass** der Ballonkatheter (1) eine mit dem mindestens einen Lichtwellenleiter (40, 41, 42) verbindbare Auswertungseinheit (6) aufweist, wobei die Auswertungseinheit (6) dazu ausgebildet ist, Licht in den mindestens einen Lichtwellenleiter (40, 41, 42) einzustrahlen und durch Auswertung von zurückgestreutem Licht eine Temperatur und/oder einen Druck zu bestimmen.

9. Ballonkatheter nach Anspruch 5 oder nach einem der Ansprüche 6 bis 8 sofern rückbezogen auf Anspruch 5, **dadurch gekennzeichnet, dass** der Ballonkatheter (1) einen im Katheterschaft (2) angeordneten Innenschaft (7) zur Aufnahme eines Führungsdrahtes des Ballonkatheters (1) aufweist, wobei sich der Innenschaft (7) durch den Balloninnenraum (30) hindurch zur Katheterspitze (5) erstreckt.

10. Ballonkatheter nach einem der vorhergehenden Ansprüche, **dadurch gekennzeichnet, dass** der Katheterschaft (2) ein mit dem Balloninnenraum (30) kommunizierendes Lumen aufweist, über das das fluide Medium zum Inflatieren des Ballons (3) in den Balloninnenraum (30) einleitbar ist.

11. Ballonkatheter nach einem der vorhergehenden Ansprüche, **dadurch gekennzeichnet, dass** der Ballonkatheter (1) mehrere Kontaktelemente (40, 41, 42) aufweist, die auf der Oberfläche (3a) des Ballons (3) angeordnet sind und zum Aufweiten der Stenose (S) dazu ausgebildet sind, bei einem inflatierten Ballon (3) im Bereich der Stenose (S) gegen die Gefäßwand (W) zu drücken, wobei das jeweilige Kontaktelement (40, 41, 42) durch einen auf der Oberfläche (3a) des Ballons (3) angeordneten Lichtwellenleiter (40, 41, 42) gebildet ist, wobei der jeweilige Lichtwellenleiter zur Temperatur- und/oder Druckmessung vorgesehen ist.

12. Ballonkatheter nach Anspruch 11, **dadurch gekennzeichnet, dass** der Ballonkatheter (1) zwei bis fünf Kontaktelemente (40, 41, 42) aufweist.

13. Ballonkatheter nach Anspruch 11 oder 12, **dadurch gekennzeichnet, dass** sich der jeweilige Lichtwellenleiter (40, 41, 42) bei einem inflatierten Ballon (3) helixförmig um den Ballon (3) herum erstreckt.
